# EUROPEAN PATENT APPLICATION

(11) **EP 4 708 209 A1**
(43) Date of publication of application: **11.03.2026**
(21) Application number: 25199509.8
(22) Date of filing: 02.09.2025
(51) Int. Cl.: G06T 19/00

(54) **HIGH-RESOLUTION HIGH-DYNAMIC-RANGE PFA MAP**

(30) Priority: 03.09.2024 US 202418823259
(71) Applicant: Biosense Webster (Israel) Ltd., 2066717 Yokneam (IL)
(72) Inventor: MAIZLIN, Doron, 2066717 Yokneam (IL); FELDMAN, Konstantin, 2066717 Yokneam (IL); BERMAN, Dror, Irvine, 92618 (US); MATALON, Naor Avidor, 2066717 Yokneam (IL); WORMS, Keren Bitton, 2066717 Yokneam (IL)
(74) Representative: Carpmaels & Ransford LLP

(57) **Abstract**

A method includes dividing a three-dimensional (3D) rendering of at least a portion of a heart into voxels having coordinates in a coordinate system of a position mapping system. Using a position tracking system, positions of one or more electrodes of a catheter are measured inside the heart during an ablation session that comprises multiple ablation instances. Each measured position is assigned a predefined number of voxels nearest to the position. A respective ablation score is calculated for each voxel, the ablation score representing at least a number of the ablation instances affecting the voxel. A respective ablation tag is assigned to each voxel and the ablation tag is graphically encoded according to the ablation score of the voxel. The graphically encoded ablation tags are overlaid on the 3D rendering to generate an ablation map. The ablation map is presented to a user.

## Description

### FIELD OF THE DISCLOSURE

This disclosure relates generally to cardiac ablation, and specifically to systems and methods for planning and monitoring of cardiac ablation using an anatomical map.

### BACKGROUND OF THE DISCLOSURE

Providing indications of ablation sites on an anatomical map of an inner wall of a cardiac chamber was previously proposed in the patent literature. For example, U.S. Patent 9,757,182 describes a method including receiving locations of multiple ablation sites formed on the surface of a heart. Distances are measured among at least some of the ablation sites based on the locations. One or more gaps between the ablation sites, which meet an alerting criterion, are identified. The identified gaps are indicated to an operator.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electroanatomical (EA) mapping and ablation system, in accordance with an example of the present disclosure;
Fig. 2 is a high-resolution, high-dynamic-range ablation map of cardiac wall tissue, in accordance with an example of the present disclosure;
Fig. 3 is a schematic illustration of a graphical user interface (GUI) used for filtering out instances of inadequate ablation from affecting the map of Fig. 2, in accordance with an example of the present disclosure; and
Fig. 4 is a flow chart that schematically illustrates a method for generating a high-resolution, high-dynamic-range ablation map, in accordance with an example of the present disclosure.

### DETAILED DESCRIPTION OF EXAMPLES

### OVERVIEW

In some cardiac catheter ablation procedures, a physician ablates tissue in a specific anatomical heart region, such as a cardiac chamber, to treat an arrhythmia. For example, the physician may ablate an entire circumference of an ostium of a pulmonary vein (PV) of a left atrium to treat atrial fibrillation (AFib).

Catheter ablation, such as one using a pulsed-field ablation (PFA) technique, may require several iterations to isolate the entire circumference of the ostium. Each iteration requires moving the ablation catheter to areas that are still insufficiently ablated or not ablated.

To successfully complete the ablation, the physician may be assisted with ablation tags overlayed on a 3D rendering of the target anatomical region of the heart, the combination called hereinafter "ablation map." The 3D rendering may be, for example, an anatomical map or an electroanatomical (EA) map (e.g., a Local Activation Time (LAT) map). The ablation tags represent surface tissue locations that were either partially or fully ablated.

Using the ablation map, the physician may try to identify non-ablated portions (known as "ablation gaps") of arrhythmogenic cardiac tissue, such as ablation gaps in the ablated tissue over the circumference of the PV ostium.

Ablation tags, however, are large icons that designate point locations where the catheter applied the ablation, which does not reflect the tissue status after ablation in detail. In particular, such an ablation map does not accurately represent the actual shapes of ablation gap regions.

The accuracy of the ablation map is further degraded as the catheter electrodes typically change position randomly during ablation due to respiration, heartbeat, or muscle contraction. The randomly changed locations of ablation hinder any single ablation tag from providing an accurate, discrete representation of the level of ablation at any given tissue location.

Examples of the present disclosure that are described hereinafter provide an algorithm to generate a high-resolution and high dynamic range ablation map that overcomes the above limitations. The disclosed ablation map is generated by overlaying small and densely spread ablation tags over a 3D rendering of a portion of the heart, such as over an anatomical map. Each tag location is assigned to a map voxel defined by the coordinate system of a position tracking system. The resulting disclosed ablation tags delineate high-resolution ablated tissue areas (e.g., with millimeter resolution) represented on the map.

An instance of ablation by an electrode affects an entire ablation zone about each voxel assigned with the measured electrode position. This ablation zone comprises a predefined number of N nearest voxels (e.g., a few tens of nearest voxels), where N is determined from a model or empirically. For example, the number and spatial configuration of assigned voxels mimic or "fill" the shape of corresponding electrode, which can vary depending on electrode shape.

During an ablation session, any voxel within an ablation zone may experience a number of ablation instances. The number of instances typically ranges from none to a maximal allowed number that can reach a few tens. The range of ablation instances (also called hereinafter "applications") defines a range of an ablation scores to be assigned to the voxel. In one case, the ablation score of a voxel defines a total level of ablation accumulated at the voxel location due to the various ablation instances. In another case the ablation score is based on a fraction of hits of a voxel out of total number of hits per instance, as defined below.

A processor that runs the disclosed algorithm calculates the ablation score for each affected voxel and graphically encodes (e.g., shades) the respective ablation tag according to the voxel's ablation score. The resulting ablation map has a high dynamic range (e.g., graded) representation of the level of ablation at the tagged voxel locations. This high dynamic range gives a detailed view of, for example, the ablation level over the circumference of an ostium.

In some examples, the disclosed ablation map is updated on-the-fly according to the progress of the ablation session. In other examples, the ablation map also considers past ablation session results by graphically encoding the ablation tags according to instances of ablation accumulated over all past ablation sessions.

In some examples, the disclosed technique provides a graphical user interface (GUI) that allows a user to filter out ablation instances of voxels if the ablation does not meet one or more conditions at the instance. An ablation instance at a voxel will thus not be scored if, for example, it was done with a too-low Touch proximity Index (TPI), done with unacceptable (e.g., too-low) electrode contact force, done at an outlier tracked position of the electrode, or done over a too-short time duration.

### SYSTEM DESCRIPTION

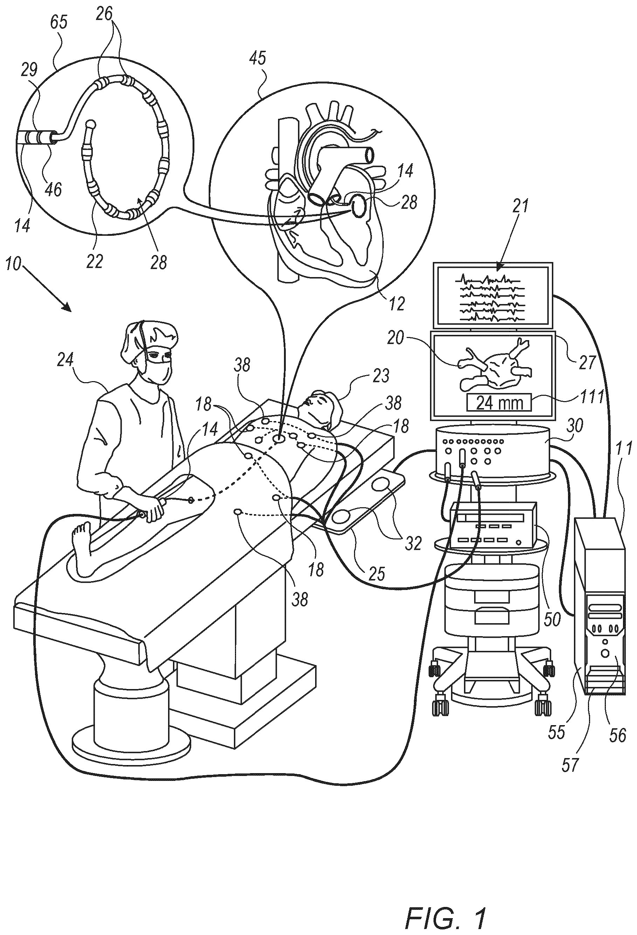

Fig. 1 is a schematic, pictorial illustration of a catheter-based electroanatomical (EA) mapping and ablation system 10, in accordance with an example of the present disclosure.

System 10 includes multiple catheters, which are percutaneously inserted by physician 24 through the patient's vascular system into a chamber or vascular structure of a heart 12 (seen in inset 45). A delivery sheath catheter is typically inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated to pacing, a catheter for sensing intracardiac electrogram signals, a catheter dedicated to ablating and/or a catheter dedicated to both EA mapping and ablating. An example catheter 14, illustrated herein, is configured for sensing bipolar electrograms and Pulsed Field Ablation (PFA). Physician 24 brings a distal tip 28 (also called hereinafter distal end assembly 28) of catheter 14 into contact with the heart wall for ablating a target site in heart 12.

As seen in inset 65, catheter 14 is an exemplary catheter that includes a lasso distal end assembly 28, including one, and preferably multiple, electrodes 26 optionally distributed over a curved spline 22. Catheter 14 may additionally include a position sensor 29, embedded in or near distal tip 28 on a shaft 46 of catheter 14, to track the position and orientation of distal tip 28. Optionally, and preferably, position sensor 29 is a magnetic-based position sensor including three magnetic coils for sensing three-dimensional (3D) position and orientation.

The magnetic-based position sensor 29 may be operated together with a location pad 25 that includes a plurality of magnetic coils 32 configured to generate magnetic fields in a predefined working volume. The real-time position of distal tip 28 of catheter 14 may be tracked based on magnetic fields generated with location pad 25 and sensed by magnetic-based position sensor 29. Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms acquired at respectively tracked cardiac tissue positions) acquired with body surface ECG electrodes 18 and intracardiac electrograms acquired with electrodes 26 of catheter 14. Recorder 11 may include pacing capability to pace the heart rhythm and/or may be electrically connected to a standalone pacer.

System 10 may include an ablation energy generator 50 adapted to conduct ablative energy to one or more electrodes at a distal tip of a catheter configured for ablation. The energy produced by ablation energy generator 50 may include, but is not limited to, radiofrequency (RF) energy or Pulse Field (PF) energy, including monopolar or bipolar and monophasic of biphasic high-voltage DC pulses, to be used to effect irreversible electroporation (IRE) or combinations thereof.

The patient interface unit (PIU) 30 is an interface configured to establish electrical communication between catheters, electrophysiological equipment, power supply, and a workstation 55 to control system 10 operation and receive EA signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, a processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO^{™} 3 System, available from Biosense Webster, Inc., 31A Technology Drive, Irvine, CA 92618.

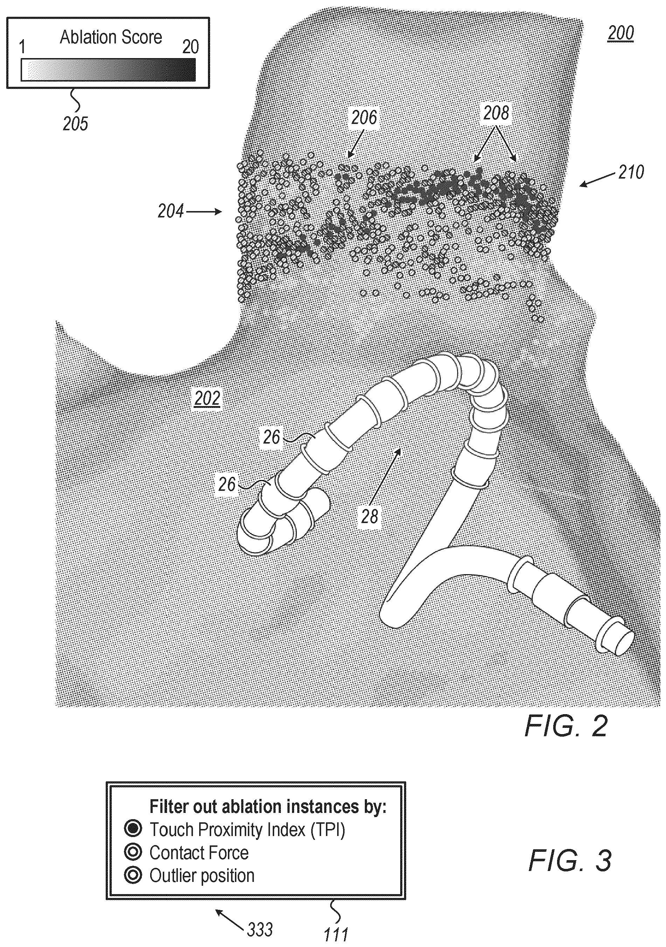

During an ablation session, processor 56 tracks the catheter electrodes' positions with a refresh rate as high as 100Hz. The result can be hundreds of ablation tags that processor 56 assigns with different ablated positions. In the disclosed example, processor 56 runs an algorithm that generates an ablation map (like map 200 of Fig. 2), including graphical encoding (e.g., coloring) of the ablation tags according to the existing level of ablation at the voxel positions represented by the ablation tags.

In some examples, processor 56 typically comprises a general-purpose computer programmed in software to carry out the functions described herein. The software may be downloaded to the computer in electronic form over a network, for example, or it may, alternatively or additionally, be provided and/or stored on non-transitory tangible media, such as magnetic, optical, or electronic memory.

This configuration of system 10 is shown by way of example to illustrate certain problems addressed by examples of the present disclosure and to demonstrate the application of these examples in enhancing the performance of such a system. However, examples of the present disclosure are by no means limited to this specific sort of example system, and the principles described herein may similarly be applied to other sorts of medical systems. For example, other multi-electrode catheter types may be used, such as a basket catheter.

### HIGH-RESOLUTION HIGH DYNAMIC RANGE PFA MAP

Fig. 2 is a high-resolution, high dynamic range ablation map 200 of cardiac wall tissue, in accordance with an example of the present disclosure. Map 200 was created by overlaying on an anatomical map 202 ablation tags (204, 206, 208) that were graphically encoded according to an ablation score 205 that counts ablation instances per voxel at an ablation session.

One example of an ablation score is based on PF applications number in every voxel. Another possible score can be based on a PF Index. In PFA, percentage of hits in every voxel is a useful criterion. For example, assuming a fraction of hits of a voxel associated to electrode position (every 16ms) during PF application (~300ms). In this example, the maximum hit number is 18, [=300/16], hit percentage is defined as the ratio of actual hits / maximum hits. This ratio is used to define percentage threshold to determine if we count this voxel in the current application.

Ablation map 200 represents a portion of a left atrium, including PV ostium 210. Ablation tags (204, 206, 208) are superimposed on map's 200 voxel locations to delineate high-resolution (e.g., with millimeter resolution) ostium 210 tissue areas represented by the anatomical map that received ablation. Each tag location is set at the center of each voxel as defined by the mapping system coordinates.

The high-resolution, high dynamic range ablation map 200 gives a clear view of the progress of the ablation, by graphically encoding the ablation tags. As seen, the dense ablation tags (204, 206, 208) show the ablation results, with ablation tags 208 having the darker tones, indicating the part of the circumference of ostium 210 that is fully ablated. Ablation tags 206 having mid tones are common in the adjacent portion of the circumference, indicating that this portion is partially ablated. Light toned ablation tags 204 indicate a less ablated portion of the ostium circumference.

Ablation map 200 is available to the user during the ablation session. The processor may update ablation map 200 according to the progress of isolating PV ostium 210 by PFA using electrodes 26 of distal end assembly 28 of catheter 14.

Fig. 3 is a schematic illustration of a graphical user interface (GUI) 111 used for filtering out instances of inadequate ablation, to avoid such from affecting map 200 of Fig. 2, in accordance with an example of the present disclosure. Using GUI 111, ablation score 205 may increase at each affected voxel only by considering an effective ablation application according to user preferences.

In the disclosed GUI, checkboxes 333 on GUI 111 allow a user to select that certain ablation instances will be filtered out, such as ablation instances at locations where touch proximity or touch force were insufficient, or electrode location was found to be an outlier. Meeting time duration is a mandatory criterion included in the algorithm.

Fig. 3 is brought by way of example. Additional or alternative filtration criteria may be considered.

### METHOD OF GENERATING HIGH-RESOLUTION, HIGH DYNAMIC RANGE ABLATION MAP

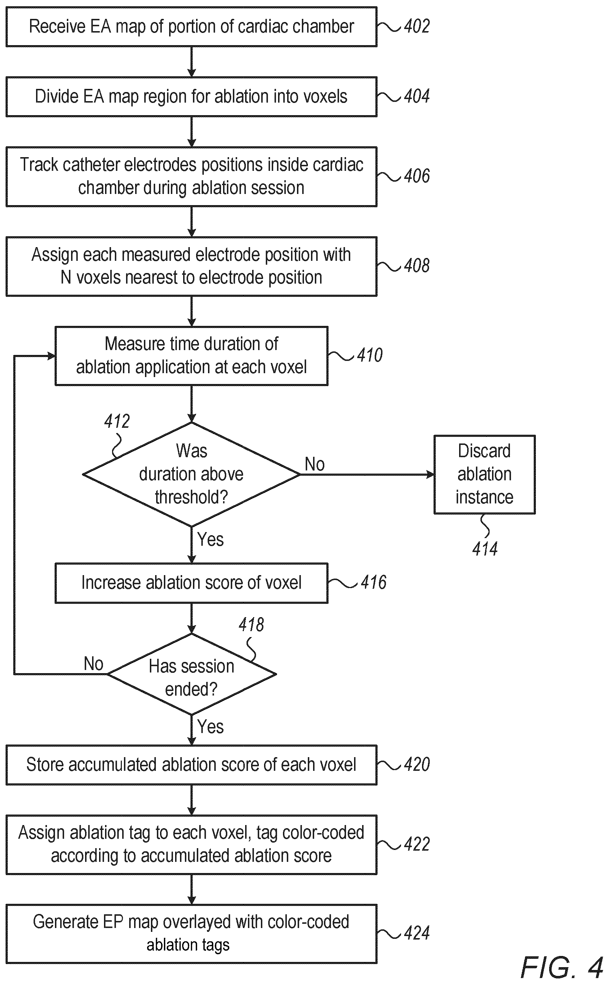

Fig. 4 is a flow chart that schematically illustrates a method for generating a high-resolution, high dynamic range ablation map 200, in accordance with an example of the present disclosure. The algorithm, according to the presented example, carries out a process that begins with processor 56 receiving an EA map of at least a portion of a cardiac chamber, in EA map receiving step 402.

In voxels definition step 404, the processor divides the EA map regions for ablation (e.g., ostium regions of a PV) into predefined voxels. The typical voxel size is 1mm³.

At location tracking step 406, the catheter's electrode position is tracked. This tracking is performed only during pulsed field ablation (PFA) applications. The processor calculates a new position after a predetermined amount of time, for example, every few tens of mSec (particularly e.g., 16 mSec), which can amount to tracking several or more positions of each electrode during one application. For a multi-electrode catheter 14, the processor simultaneously tracks positions of multiple electrodes.

At voxels assignment step 408, each tracked electrode position is assigned with N voxels (e.g., N=19 in a cubic grid) nearest to the electrode position. N can vary based on the grid used. For example, a tetrahedronal grid or a hexagonal pyramid grid may be used.

At ablation application step 410, a processor running the PFA application (e.g., instances) commands a generator to continuously deliver PF energy, typically for 300-400ms. A PF session includes PF generator running a predefined sequence of PFA applications, typically 12-24 applications (e.g., instances), with a one-second interval between applications. To avoid tracking energizing an electrode in poor contact with tissue (e.g., in the blood), the system may gate ablation with TPI being above a predefined threshold.

At a time duration checking step 412, the processor checks if the time duration of the PF instance at any of the N voxels about a given electrode location was above a threshold time duration (e.g., above 25 mSec). Another threshold criterion can be a minimal percentage of hits in a voxel during one application.

If the answer is No, the processor discards the instance from consideration for that voxel's ablation score, at an ablation instance discarding step 414.

For voxels that received ablation over a time duration above the threshold, the processor increases the ablation score 205 by one count, at ablation score counting step 416. At session checking step 418, the processor checks if the session has ended and if it has not, the processor repeats steps 410-416.

Once the ablation session ends, at the ablation scores storing step 420, the processor stores the aggregated (e.g., accumulated) ablation score of each voxel at that session.

At ablation tags generation step 422, processor 56 assigns an ablation tag to each of the scored voxels; the tag is graphically encoded (for example, color-coded) according to the accumulated ablation score of each voxel. If an ablation score already exists for that voxel due to prior ablations, the processor selects the highest score of the voxel among all scores to graphically encode (color-code) the ablation tag.

Finally, at ablation map generation step 424, the processor overlays the graphically encoded (color-coded) ablation tags on the EP map to generate an ablation map such as map 200.

After generating the ablation map, processor 56 can present it to a user on display device 27 or store it in memory 57.

With the generated ablation map, it may be easier (e.g., for the user) to identify non-ablated or less-ablated portions, i.e., "ablation gaps", of the ablated tissue, by analyzing the graphically encoded (e.g., color coded) high-resolution voxel ablation tags. For example, the user may identify locations with brighter color as locations where additional ablation may be required, and direct future ablations to that site.

The flow chart of Fig. 4 is provided as an example. In other examples, the process may include different steps, such as including more voxel filtering-out steps rather than letting the user select if to activate such using GUI 111 of Fig. 3. The processor may receive any other 3D rendering of the cardiac chamber.

### EXAMPLES

### Example 1

A method includes dividing a three-dimensional (3D) rendering (202) of at least a portion of a heart into voxels having coordinates in a coordinate system of a position mapping system (10). Using a position tracking system, positions of one or more electrodes (26) of a catheter are measured inside the heart during an ablation session that comprises multiple ablation instances. Each measured position is assigned a predefined number of voxels nearest to the position. A respective ablation score (205) is calculated for each voxel, the ablation score representing at least a number of the ablation instances affecting the voxel. A respective ablation tag (204, 206, 208) is assigned to each voxel and the ablation tag is graphically encoded according to the ablation score (205) of the voxel. The graphically encoded ablation tags (204, 206, 208) are overlaid on the 3D rendering (202) to generate an ablation map (200). The ablation map (200) is presented to a user.

### Example 2

The method according to example 1, wherein calculating the ablation score (205) comprises including an ablation instance in the ablation score (205) of a voxel only if a time duration of ablation at the ablation instance exceeds a predefined threshold.

### Example 3

The method according to example 1, wherein calculating the ablation score (205) comprises including an ablation instance in the ablation score (205) of a voxel only if hit percentage at the ablation instance exceeds a predefined threshold.

### Example 4

The method according to any of examples 1 through 3, wherein assigning the predefined number of voxels nearest to the position comprises assigning the nearest voxels as defined on one of a cubic grid, a tetrahedral grid, and a hexagonal pyramid grid.

### Example 5

The method according to any of examples 1 through 4, wherein graphically encoding the ablation tag (204, 206, 208) comprises coloring the ablation tag (204, 206, 208) with a shade of a color representing the ablation score (205).

### Example 6

The method according to any of examples 1 through 5, and comprising providing a graphical user interface (GUI) (111) configured to allow (333) the user to omit an ablation instance from calculation of the ablation score (205) based on at least one of insufficient electrode contact force and an exceedingly low touch proximity index (TPI).

### Example 7

The method according to claim 1, wherein the catheter is a multi-electrode catheter (14).

### Example 8

The method according to claim 1, wherein the portion of the heart is a cardiac chamber.

### Example 9

A system comprising a display device (27), and a processor (56), which is configured to (i) divide a three-dimensional (3D) rendering (202) of at least a portion of a heart into voxels having coordinates in a coordinate system of a position mapping system (10), (ii) using a position tracking system, measure positions of one or more electrodes (26) of a catheter inside the heart during an ablation session that comprises multiple ablation instances, (iii) assign each measured position with a predefined number of voxels nearest to the position, (iv) calculate a respective ablation score (205) for each voxel, the ablation score representing at least a number of the ablation instances affecting the voxel, (v) assign a respective ablation tag (204, 206, 208) to each voxel and graphically encode the ablation tag according to the ablation score (205) of the voxel, (vi) overlay the graphically encoded ablation tags (204, 206, 208) on the 3D rendering (202) to generate an ablation map (200), and (vii) present the ablation map (200) to a user on the display device (27).

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A method, comprising:
dividing a three-dimensional (3D) rendering of at least a portion of a heart into voxels having coordinates in a coordinate system of a position mapping system;
using a position tracking system, measuring positions of one or more electrodes of a catheter inside the heart during an ablation session that comprises multiple ablation instances;
assigning each measured position with a predefined number of voxels nearest to the position;
calculating a respective ablation score for each voxel, the ablation score representing at least a number of the ablation instances affecting the voxel;
assigning a respective ablation tag to each voxel and graphically encoding the ablation tag according to the ablation score of the voxel;
overlaying the graphically encoded ablation tags on the 3D rendering to generate an ablation map; and
presenting the ablation map to a user.

2. The method according to claim 1, wherein calculating the ablation score comprises including an ablation instance in the ablation score of a voxel only if a time duration of ablation at the ablation instance exceeds a predefined threshold.

3. The method according to claim 1, wherein calculating the ablation score comprises including an ablation instance in the ablation score of a voxel only if hit percentage at the ablation instance exceeds a predefined threshold.

4. The method according to claim 1, wherein assigning the predefined number of voxels nearest to the position comprises assigning the nearest voxels as defined on one of a cubic grid, a tetrahedral grid, and a hexagonal pyramid grid.

5. The method according to claim 1, wherein graphically encoding the ablation tag comprises coloring the ablation tag with a shade of a color representing the ablation score.

6. The method according to claim 1, and comprising providing a graphical user interface (GUI) configured to allow the user to omit an ablation instance from calculation of the ablation score based on at least one of insufficient electrode contact force and an exceedingly low touch proximity index (TPI).

7. The method according to claim 1, wherein the catheter is a multi-electrode catheter.

8. The method according to claim 1, wherein the portion of the heart is a cardiac chamber.

9. A system, comprising:
a display device; and
a processor, which is configured to:
divide a three-dimensional (3D) rendering of at least a portion of a heart into voxels having coordinates in a coordinate system of a position mapping system;
using a position tracking system, measure positions of one or more electrodes of a catheter inside the heart during an ablation session that comprises multiple ablation instances;
assign each measured position with a predefined number of voxels nearest to the position;
calculate a respective ablation score for each voxel, the ablation score representing at least a number of the ablation instances affecting the voxel;
assign a respective ablation tag to each voxel and graphically encode the ablation tag according to the ablation score of the voxel;
overlay the graphically encoded ablation tags on the 3D rendering to generate an ablation map; and
present the ablation map to a user on the display device.

10. The system according to claim 9, wherein the processor is configured to calculate the ablation score by including an ablation instance in the ablation score of a voxel only if a time duration of ablation at the ablation instance exceeds a predefined threshold.

11. The system according to claim 9, wherein the processor is configured to calculate the ablation score by including an ablation instance in the ablation score of a voxel only if hit percentage at the ablation instance exceeds a predefined threshold.

12. The system according to claim 9, wherein the processor is configured to assign the predefined number of voxels nearest to the position by assigning the nearest voxels as defined on one of a cubic grid, a tetrahedral grid, and a hexagonal pyramid grid.

13. The system according to claim 9, wherein the processor is configured to graphically encode the ablation tag by coloring the ablation tag with a shade of a color representing the ablation score.

14. The system according to claim 9, wherein the processor is further configured to provide a graphical user interface (GUI) configured to allow the user to omit an ablation instance from calculation of the ablation score based on at least one of insufficient electrode contact force and an exceedingly low touch proximity index (TPI).

15. The system according to claim 9, wherein the catheter is a multi-electrode catheter and wherein the portion of the heart is a cardiac chamber.
